# EUROPÄISCHE PATENTSCHRIFT

(11) **EP 0 588 839 B1**
(45) Veröffentlichungstag und Bekanntmachung des Hinweises auf die Patenterteilung: **16.08.1995**
(21) Anmeldenummer: 92911100.3
(22) Anmeldetag: 25.05.1992
(51) Int. Cl.: A61K 31/21, A61K 9/70

(54) **NITROGLYCERIN-PFLASTER UND VERFAHREN ZU SEINER HERSTELLUNG**
NITROGLYCERINE PATCH AND PROCESS FOR MAKING IT
EMPLATRE A LA NITROGLYCERINE ET SON PROCEDE DE FABRICATION

(30) Priorität: 10.06.1991 DE 4118891
(43) Veröffentlichungstag der Anmeldung: 30.03.1994
(73) Patentinhaber: SCHWARZ PHARMA AG, D-40789 Monheim (DE); LTS Lohmann Therapie-Systeme GmbH & Co. KG, 56513 Neuwied (DE)
(72) Erfinder: BEUTNER, Dieter, D-4019 Monheim (DE); KNOBELSDORFF, V., Henning, D-5300 Bonn 3 (DE); WOLFF, Hans-Michael, D-4019 Monheim (DE); HOFFMANN, Rainer, D-5450 Neuwied 22 (DE); MECONI, Reinhold, D-5450 Neuwied 11 (DE); KLEIN, Robert, Peter, D-5450 Neuwied 11 (DE)
(74) Vertreter: Cohausz & Florack Patentanwälte
(86) Internationale Anmeldenummer: EP9201169
(87) Internationale Veröffentlichungsnummer: WO9222292

(56) Entgegenhaltungen:
- EP-A- 0 062 682
- EP-A- 0 272 562
- EP-A- 0 285 550
- EP-A- 0 427 877
- EP-A- 0 435 199
- EP-A- 0 450 986
- WO-A-86/06281
- GB-A- 2 086 224
- US-A- 4 814 168
- Derwent File Supplier & Base WPIL, AN=88-033094 [05], Derwent Publications Ltd, London, GB, & JP,A,62292877 (NIPPON SHOKUBAI KAGAKU) 19 December 1987, siehe Zusammenfassung
- Chemical Abstracts, Band 97, Nr. 12, September 1982, (Columbus, Ohio, US), siehe Seite 403, Zusammenfassung Nr. 98386f, & JP,A,82107155 (NITTO ELECTRIC INDUSTRIAL CO., LTD) 3. Juli 1982, siehe Zusammenfassung

## Beschreibung

Die vorliegende Erfindung betrifft ein Hautpflaster zur transdermalen Verabreichung von Nitroglycerin, bestehend aus einer Trägerfolie und einer Nitroglycerin enthaltenden Klebemasse auf Basis eines vernetzten Acrylat-Copolymerisates. Das Pflaster weist weiterhin eine Schutzfolie auf, die vor Gebrauch des Pflasters, d.h. vor Anbringen desselben auf die Haut durch Anziehen entfernt wird.

Hautpflaster zur transdermalen Verabreichung von Nitroglycerin sind zahlreich bekannt. Zum Beispiel beschreiben DE 2135533 und DE 3315272 Pflaster, die mehrschichtig aufgebaut sind und die Wirkstoffabgabe steuern. Nitroglycerin wird nach verschiedenen Mechanismen, sei es aus einem einschichtigen Reservoir durch eine Steuermembran (DE 2135533), sei es durch besondere Gestaltung des mehrschichtigen Reservoirs (DE 3315272), freigesetzt. Da die vielschichtigen Hautpflaster insbesondere in ihrer Herstellung recht teuer sind, hat man in jüngerer Vergangenheit Pflaster entwickelt, die neben der Trägerfolie aus einer einzigen Schicht aufgebaut sind. Um in genügendem Ausmaß Nitroglycerin aufnehmen und wieder in genügendem Maß Nitroglycerin an die Haut abgeben zu können, hat man hierbei verschiedene selbstklebende Haftklebemassen mit den verschiedensten Eigenschaften in bezug auf Wirkstoffaufnahmekapazität, Wirkstoffabgabe und Haftfähigkeit auf der Haut entwickelt. Als Beispiele hierfür seien genannt GB-A 2095108, DE-OS 3231400, GB-A 2086224, EP-A 0062682, EP 85903926.5, EP 86902978.5, EP 0285550, EP 0272562, US 4608249 und DE-PS 3200369. Je nach den eingesetzten Materialien und dem Vernetzungsgrad haben die Pflaster unterschiedliche Aufnahmekapazität und Abgabefähigkeit für Nitroglycerin und sind durch eine unterschiedliche Haftfähigkeit zur Haut gekennzeichnet. Unterschiedliche Hautverträglichkeit spielt ebenso eine erhebliche Rolle. Manche der Pflaster enthalten zusätzlich Stoffe zur Erhöhung des transepidermalen Stofftransports (sog. Resorptionsbeschleuniger).

Aufgabe der vorliegenden Erfindung ist die Schaffung eines Hautpflasters zur transdermalen Verabreichung von Nitroglycerin, das gekennzeichnet ist durch Einsatz eines Haftklebers, der nicht nur eine möglichst hohe Aufnahmekapazität, sondern auch eine hohe Abgabefähigkeit für Nitroglycerin besitzt, so daß für die notwendige Freisetzungsmenge pro Tag die Freisetzungsfläche des Pflaster klein gehalten werden kann und hierdurch die Kosten des Pflasters möglichst niedrig sind. Gleichzeitig soll durch Einsetzung eines speziellen Klebers das Herstellungsverfahren vereinfacht, seine Kosten gering und der Zusatz von Resorptionsbeschleunigern eingespart werden. Diese Vereinfachung der pharmazeutischen Formulierung verringert zugleich das Risiko von Hautirritationen und/oder einer unkontrollierbaren Veränderung der Nitroglycerinkonzentration in der Haftklebemasse, was mit der Penetration von Resorptionsbeschleunigern aus der Klebemasse in die Haut einhergehen kann.

Das erfindungsgemäße Hautpflaster zur transdermalen Verabreichung von Nitroglycerin, bestehend aus einer Trägerfolie, einer Nitroglycerin enthaltenden Klebemasse auf Basis eines vernetzten Acrylat-Vinylacetat-Copolymerisats und einer üblichen abziehbaren Schutzfolie ist dadurch gekennzeichnet, daß die das Nitroglycerin enthaltende Klebemasse dadurch erhalten ist, daß in einer ersten Stufe ein Gemisch aus 21 bis 40 Gew.-% Vinylacetat, 55 bis 70 Gew.-% eines Acrylsäure-C₂₋₈-alkylesters und 3 bis 10 Gew.-% eines Acrylsäure-C₂₋₄-hydroxyacrylesters, bei 100 Gew.-% Monomeren im Gemisch, in einem organischen Lösungsmittel einer radikalischen Polymerisation unterworfen wird, sodann in einer zweiten Stufe ein übliches Vernetzungsmittel in einem organischen Lösungsmittel und das Nitroglycerin in der für die beabsichtigte Anwendung des Pflasters notwendigen Menge, gegebenenfalls in einem organischen Lösungsmittel zugemischt wird und schließlich in einer dritten Stufe das erhaltene Gemisch bzw. das bestimmte Acrylat-Vinylacetat-Copolymerisat in einer zusätzlichen Stufe unter Erwärmen und Entfernen des eingesetzten organischen Lösungsmittels bzw. Lösungsmittelgemischs vernetzt wird und das enthaltene Nitroglycerin durch die nachträgliche und zusätzliche Vernetzung des besonderen Acrylat-Vinylacetat-Copolymerisats in besonderer Weise in die Klebemasse "eingebaut" wird. Das Acrylat-Vinylacetat-Copolymerisat hat eine relative Viskosität von 3,0 bis 4,2.

Bevorzugt enthält das Monomerengemisch neben Vinylacetat 2-Ethylhexylacrylat und Hydroxyethylacrylat. Bevorzugt ist die nachfolgende Vernetzung des besonderen Acrylat-Vinylacetat-Copolymerisats mit einem Titansäureester bestehend aus Polybutyltitanat und/oder Titanacetylacetonat, insbesondere in einer Menge von 0,3 bis 3 Gew.-% hiervon, die Gewichtsprozente bezogen auf das Gewicht des Copolymerisats, durchgeführt.

Das Verfahren zur Herstellung des erfindungsgemäßen Pflasters ist dadurch gekennzeichnet, daß eine Nitroglycerin in der für die beabsichtigte Anwendung des Pflasters notwendigen Menge und einen üblichen Vernetzer oder ein übliches Vernetzergemisch enthaltende Lösung eines durch radikalische Polymerisation eines Monomerengemisches bestehend aus 21 bis 40 Gew.-% Vinylacetat, 55 bis 70 Gew.-% eines Acrylsäure-C₂₋₈-alkylesters und 3 bis 10 Gew.-% eines Acrylsäure-C₂₋₄-hydroxyalkylesters erhaltenen Copolymerisats auf die Schutzfolie des Pflasters in der gewünschten Schichtdicke aufgetragen wird und das Lösungsmittel oder Lösungsmittelgemisch unter Erwärmen entfernt und so die zusätzliche Vernetzung des besonderen Acrylat-Vinylacetat-Copolymerisats durchgeführt wird.

Vorzugsweise ist das Verfahren dadurch gekennzeichnet, daß das Acrylat-Vinylacetat-Copolymerisat, Nitroglycerin und Vernetzer gelöst sind in einem Lösungsmittel, das 20 bis 40 Gew.-% Ethanol oder eines Ethanol-Methanol-Gemisches enthält, mit einem Feststoffanteil, bestehend aus 40 bis 60 Gew.-% des Gemischs aus dem besonderen Acrylat-Vinylacetat-Copolymerisat, Vernetzer und dem Nitroglycerin.

### Ausführungsbeispiel

Herstellungsverfahren für Hautpflaster zur transdermalen Anwendung von Nitroglycerin gemäß vorliegender Erfindung, die Mengenangaben bezogen auf eine Ansatzgröße von 100 m².

Zu 16,00 kg einer 40 %-igen Lösung (G/G) des Acrylat-Vinylacetat-Copolymerisates werden unter intensiver Durchmischung 4,00 kg Nitroglycerin in öliger Form langsam zugeführt. Anschließend wird die Mischung durch Rühren homogenisiert. Es resultiert eine 20 %-ige (G/G) Lösung von Nitroglycerin in dieser Kleberlösung.

Das Acrylat-Vinylacetat-Copolymerisat wird wie folgt hergestellt:
Von der Gesamtmenge von 112 g Vinylacetat, 270 g 2-Ethylhexylacrylat, 20 g Hydroxyethylacrylat, 1,4 g Azodiisobutyronitril und 407 g Ethylacetat werden 112 g Vinylacetat, 39 g 2-Ethylhexylacrylat, 3 g Hydroxyethylacrylat und 0,5 g Azodiisobutyronitril zu 115 g Ethylacetat zugegeben und bis zum Rückfluß erhitzt. Der Restanteil der Bestandteile wird über eine Zeitdauer von 4 Stunden unter konstantem Rückfluß zugegeben. Nach Beendigung der Polymerisation wird die Mischung auf Raumtemperatur abgekühlt. Die resultierende Kleberpolymerlösung hat eine Viskosität von 5300 mPa.s bei 25°C, gemessen mit einem Brookfield-Viskometer, einen Feststoffanteil von 47,9 % und die relative Viskosität beträgt 3,1.

Zu dieser Lösung werden 1,35 g Titanacetylacetonat und genügend Ethanol oder Ethanol-Ethylacetat-Mischung zugegeben, um den Feststoffgehalt im Produkt auf 40 % einzustellen.

### Beipiel 1

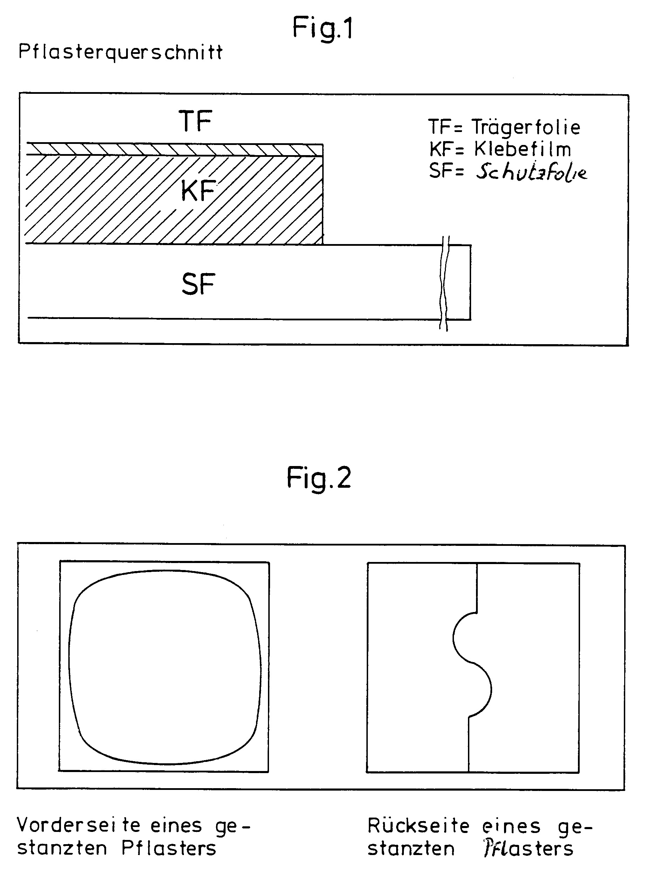

Die oben genannte 20 % (G/G) Nitroglycerin enthaltende Kleberlösung wird auf eine 100 »m dicke silikonisierte Polyesterfolie aufgetragen, so daß nach dem Entfernen des Lösungsmittels ein Film mit einem Flächengewicht von 92 g/m² resultiert. Dieser Film wird mit einer 19 »m dicken Polyesterfolie abgedeckt und zu Pflastern mit einer Kontaktfläche von 16 qcm gestanzt (Abb. 1 und 2). Ein so hergestelltes Hautpflaster mit einem Gewicht von 420 mg enthält 55 mg Nitroglycerin.

Zur Beurteilung des Wirkstoffliberationsverhaltens in vitro wird ein Pflaster mit einer ausgestanzten Fläche von 3,14 qcm in einer modifizierten Franz-Diffusionszelle (vgl. Chien, Yie W., Drugs of Today Vol. 23, No. 11 (1987) 625 - 646) auf einer Kautpräparation haarloser Mäuse befestigt.

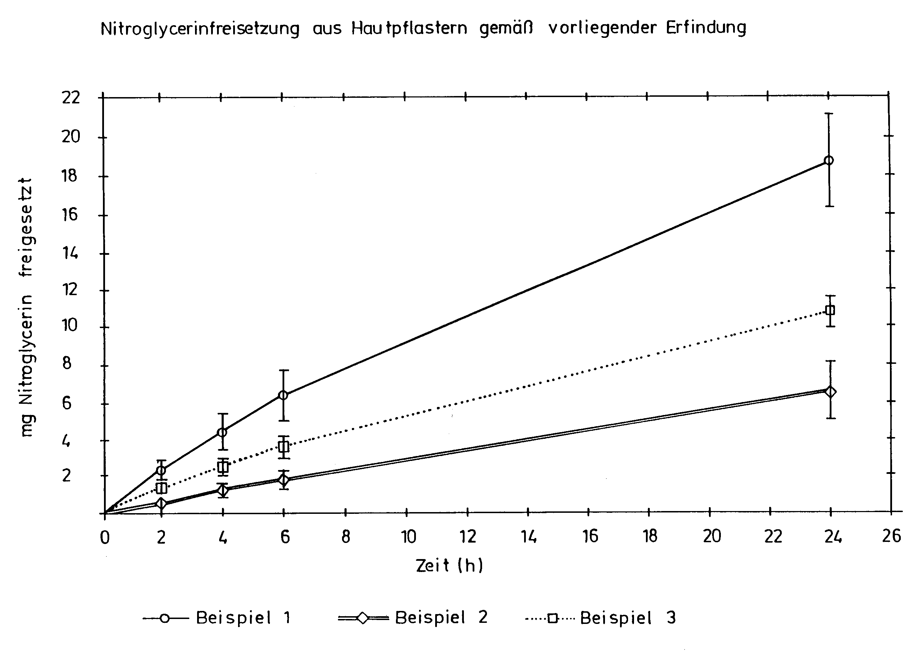

Unmittelbar anschließend wird die Zelle mit 18,00 ml isotonischer Phosphatpufferlösung (32 ± 0,5°C) luftblasenfrei befüllt und auf 32 ± 0,5°C thermostatisiert. Nach 2, 4, 6 und 24 Stunden wird das Freisetzungsmedium durch frische auf 32 ± 0,5°C thermostatisierte Lösung ersetzt. Die entnommene Lösung wird HPLC-chromatographisch (= hochleistungsflüssigkeitschromatographisch (Lit.: Pharm.Biol. 4, 32 (1981)) auf ihren Nitroglyceringehalt untersucht. Das nach dieser Methode gemessene Freisetzungsprofil für ein 16 qcm großes Pflaster ist in Abb. 3 wiedergegeben.

Die mittleren Nitroglycerin-Freisetzungsraten (± S.D.) betrugen (n = 3):
nach 2 Stunden 2,32 ± 0,56 mg/16 qcm
nach 4 Stunden 4,42 ± 1,00 mg/16 qcm
nach 6 Stunden 6,43 ± 1,33 mg/16 qcm
nach 24 Stunden 18,74 ± 2,43 mg/16 qcm

### Beispiel 2

Der oben genannten 20 % (G/G) Nitroglycerin enthaltenden Kleberlösung werden zusätzlich 0,8 % (G/G) Titanacetylacetonat (Hersteller: Dynamit Nobel Nederland B.V., 75 %-ige (G/G) Lösung in Isopropanol), bezogen auf einen 40 %-igen (G/G) Feststoffanteil der Polyacrylatkleberlösung zuzüglich Nitroglycerin, zugesetzt und das Gemisch homogenisiert. Diese Lösung wird auf eine 100 »m dicke silikonisierte Polyesterfolie aufgetragen, so daß nach dem Entfernen des Lösungsmittels ein Film mit einem Flächengewicht von 93 g/m² resultiert. Dieser Film wird mit einer 19 »m dicken Polyesterfolie abgedeckt und zu Pflastern mit einer Kontaktfläche von 16 qcm gestanzt (Abb. 1 und 2). Ein so hergestelltes Hautpflaster mit einem Gewicht von 420 mg enthält 55 mg Nitroglycerin.

Die Wirkstofffreisetzung in vitro wurde entsprechend der Methode in Beispiel 1 durchgeführt. Das entsprechende Freisetzungsprofil ist ebenfalls in Abb. 3 graphisch wiedergegeben.

Die mittleren Nitroglycerin-Freisetzungsraten (± S.D.) betrugen (n = 3):
nach 2 Stunden 0,54 ± 0,20 mg/16 qcm
nach 4 Stunden 1,20 ± 0,37 mg/16 qcm
nach 6 Stunden 1,78 ± 0,53 mg/16 qcm
nach 24 Stunden 6,60 ± 1,56 mg/16 qcm

### Beispiel 3

Die oben genannte 20 % (G/G) Nitroglycerin enthaltende Kleberlösung wird auf eine 100 »m dicke silikonisierte Polyesterfolie aufgetragen, so daß nach dem Entfernen des Lösungsmittels ein Film mit einem Flächengewicht von 64 g/m² resultiert. Dieser Film wird mit einer 19 »m dicken Polyesterfolie abgedeckt und zu Pflastern mit einer Kontaktfläche von 16 qcm gestanzt (Abb. 1 und 2). Ein so hergestelltes Hautpflaster mit einem Gewicht von 360 mg enthält 40 mg Nitroglycerin. Die Wirkstofffreisetzung in vitro wurde entsprechend der Methode in Beispiel 1 durchgeführt. Das entsprechende Freisetzungsprofil ist ebenfalls in Abb. 3 graphisch wiedergegeben.

Die mittleren Nitroglycerin-Freisetzungsraten (± S.D.) betrugen (n = 3):
nach 2 Stunden 1,27 ± 0,29 mg/16 qcm
nach 4 Stunden 2,48 ± 0,48 mg/16 qcm
nach 6 Stunden 3,56 ± 0,60 mg/16 qcm
nach 24 Stunden 10,79 ± 0,82 mg/16 qcm.

## Patentansprüche

1. Hautpflaster zur transdermalen Verabreichung von Nitroglycerin, bestehend aus einer Trägerfolie, einer Nitroglycerin enthaltenden Klebemasse auf Basis eines Acrylat-Vinylacetat-Copolymerisats und einer vor Gebrauch entfernbaren üblichen Schutzfolie, **dadurch gekennzeichnet,** daß die das Nitroglycerin enthaltende Klebemasse hergestellt ist durch:
1) radikalische Polymerisation eines Gemisches von 21 bis 40 Gew.-% Vinylacetat, 55 bis 70 Gew.-% eines Acrylsäure-C₂₋₈-alkylesters und 3 bis 10 Gew.-% eines Acrylsäure-C₂₋₄-hydroxyalkylesters, bezogen auf 100 Gew.-% des eingesetzten Monomerengemisches, in einem organischen Lösungsmittel,
2) Zumischen eines üblichen Vernetzers in einem organischen Lösungsmittel und des Nitroglycerins in der für die Anwendung des Pflasters notwendigen Menge und
3) Vernetzung des erhaltenen Gemischs unter Erwärmung und Entfernung des eingesetzten Lösungsmittels oder Lösungsmittelgemischs.

2. Hautpflaster gemäß Anspruch 1, dadurch gekennzeichnet, daß der Acrylsäure-C₂₋₄-hydroxyalkylester Hydroxyethylacrylat ist.

3. Hautpflaster gemäß einem der Ansprüche 1 und 2, dadurch gekennzeichnet, daß der Acrylsäure-C₂₋₈-alkylester neben 2-Hydroxyethylacrylat Ethylacrylat ist.

4. Hautpflaster gemäß einem der Ansprüche 1 bis 3, dadurch gekennzeichnet, daß der Vernetzer ein Titansäureester oder ein Titansäureestergemisch ist.

5. Hautpflaster gemäß Anspruch 4, dadurch gekennzeichnet, daß zur Vernetzung 0,3 bis 3 Gew.-% eines Titansäureesters oder Titansäureestergemischs eingesetzt werden, wobei die Gewichtsprozente bezogen sind auf das Gewicht des Vernetzer enthaltenden, durch radikalische Polymerisation erhaltenen Copolymerisats.

6. Hautpflaster gemäß einem der Ansprüche 4 und 5, dadurch gekennzeichnet, daß der Vernetzer Titanacetylacetonat und/oder Polybutyltitanat ist.

7. Verfahren zur Herstellung eines Pflasters gemäß einem der Ansprüche 1 bis 6, dadurch gekennzeichnet, daß ein das Nitroglycerin in der zur Anwendung des Pflasters notwendigen Menge und einen Vernetzer enthaltende Lösung eines Acrylat-Vinylacetat- Copolymerisats, hergestellt durch radikalische Polymerisation eines Monomerengemisches bestehend aus 21 bis 40 Gew.-% Vinylacetat, 55 bis 70 Gew.-% eines Acrylsäure-C₂₋₈-alkylesters und 3 bis 10 Gew.-% eines Acrylsäure-C₂₋₄-hydroxyalkylesters, bezogen auf 100 Gew.-% des eingesetzten Monomerengemisches, in einem organischen Lösungsmittel, auf die Schutzfolie des Pflasters in der gewünschten Schichtdicke aufgetragen wird, das Lösungsmittel oder Lösungsmittelgemisch unter Erwärmen entfernt wird, und so die Vernetzung des besonderen Acrylat-Vinylacetat-Copolymerisats durchgeführt wird und sodann die Trägerfolie aufgebracht wird und das Pflaster auf die gewünschte Größe zugeschnitten und/oder gestanzt wird.

8. Verfahren gemäß Anspruch 7, dadurch gekennzeichnet, daß das besondere Acrylat-Vinylacetat-Copolymerisat, das Nitroglycerin und der Vernetzer gemeinsam in einem Lösungsmittelgemisch gelöst sind, das 20 bis 40 Gew.-% Ethanol oder eines Ethanol-Methanol-Gemisches enthält, und sein Feststoffanteil, bestehend aus dem besonderen Acrylat-Vinylacetat-Copolymerisat, Vernetzer und Nitroglycerin, 40 bis 60 Gew.-% beträgt.

## Claims

1. A dermal patch for the transdermal administration of nitroglycerine, consisting of a carrier film, an adhesive material containing nitroglycerine and based on an acrylate-vinyl acetate copolymer, and a customary protective film which can be removed before use, characterised in that the adhesive material containing nitroglycerine is produced by:
1) radical polymerisation of a mixture of 21 to 40 % by weight vinyl acetate, 55 to 70 % by weight of an acrylic acid C₂₋₈ alkyl ester and 3 to 10 % by weight of an acrylic acid C₂₋₄ hydroxyalkyl ester, based on 100 % by weight of the monomer mixture used, in an organic solvent,
2) admixture of a customary crosslinking agent in an organic solvent with the nitroglycerine in the amount necessary for the application of the patch, and
3) crosslinking the mixture obtained by heating and removal of the solvent or solvent mixture used.

2. A dermal patch according to claim 1, characterised in that the acrylic acid C₂₋₄ hydroxyalkyl ester is hydroxyethyl acrylate.

3. A dermal patch according to one of claims 1 and 2, characterised in that the acrylic acid C₂₋₈ alkyl ester in addition to 2-hydroxyethyl acrylate is ethyl acrylate.

4. A dermal patch according to any one of claims 1 to 3, characterised in that the crosslinking agent is a titanic acid ester or a titanic acid ester mixture.

5. A dermal patch according to claim 4, characterised in that 0.3 to 3 % by weight of a titanic acid ester or titanic acid ester mixture is used for crosslinking, wherein the percentages by weight are based on the weight of the copolymer obtained by radical polymerisation and containing the crosslinking agent.

6. A dermal patch according to one of claims 4 and 5, characterised in that the crosslinking agent is titanium acetylacetonate and/or polybutyl titanate.

7. A process for producing a patch according to any one of claims 1 to 6, characterised in that a solution of an acrylate-vinyl acetate copolymer containing nitroglycerine in the amount necessary for the application of the patch and a crosslinking agent is produced by radical polymerisation of a monomer mixture consisting of 21 to 40 % by weight of vinyl acetate, 55 to 70 % by weight of an acrylic acid C₂₋₈ alkyl ester and 3 to 10 % by weight of an acrylic acid C₂₋₄ hydroxyalkyl ester, based on 100 % by weight of the monomer mixture used, in an organic solvent, is applied at the desired layer thickness to the protective film of the patch, the solvent or solvent mixture is removed by heating and thus crosslinking is effected of the special acrylate-vinyl acetate copolymer and then the carrier film is applied and the patch is cut out and/or punched out to the desired size.

8. A process according to claim 7, characterised in that the special acrylate-vinyl acetate monomer, the nitroglycerine and the crosslinking agent are dissolved in a solvent mixture which contains 20 to 40 % by weight of ethanol or of an ethanol-methanol mixture, and the solids content of which, consisting of the special acrylate-vinyl acetate copolymer, the crosslinking agent and nitroglycerine, is 40 to 60 % by weight.

## Revendications

1. Pansement cutané pour l'administration transdermique de nitroglycérine, composé d'une feuille support, d'une masse adhésive contenant de la nitroglycérine à base d'un copolymère réticulé acrylate-acétate de vinyle, et d'une feuille protectrice à retirer avant emploi, ***caractérisé en ce que*** la masse adhésive contenant de la nitroglycérine est fabriquée par :
1) polymérisation radicalaire d'un mélange de 21 à 40 % en poids d'acétate de vinyle, de 55 à 70 % en poids d'un alkylester en C₂₋₈ de l'acide acrylique et de 3 à 10 % en poids d'un hydroxyalkylester en C₂₋₄ de l'acide acrylique, rapportés à 100 % en poids de monomères dans le mélange, dans un solvant organique,
2) ajout d'un agent de réticulation conventionnel dans un solvant organique et de la nitroglycérine dans la quantité nécessaire pour l'utilisation envisagée pour le pansement, et
3) réticulation du mélange obtenu par réchauffement et élimination du solvant ou du mélange de solvants employé.

2. Pansement cutané selon la revendication 1, ***caractérisé en ce que*** l'hydroxyalkylester en C₂₋₄ de l'acide acrylique est l'hydroxyéthylacrylate.

3. Pansement cutané selon l'une des Revendications 1 et 2, ***caractérisé en ce que*** l'alkylester en C₂₋₈ de l'acide acrylique est, outre le 2-hydroxyéthylacrylate, l'éthylacrylate.

4. Pansement cutané selon l'une des Revendications 1 à 3, ***caractérisé en ce que*** l'agent de réticulation est un ester de l'acide titanique ou un mélange d'esters de l'acide titanique.

5. Pansement cutané selon la Revendication 4, ***caractérisé en ce qu***'on utilise pour la réticulation, 0,3 à 3 % d'un ester de l'acide titanique ou d'un mélange d'esters de l'acide titanique, les pourcentages en poids étant rapportés au poids du copolymère obtenu par polymérisation radicalaire et contenant l'agent de réticulation.

6. Pansement cutané selon l'une des Revendications 4 et 5, ***caractérisé en ce que*** l'agent de réticulation est l'acétylacétonate de titane et/ou le polybutyltitanate.

7. Procédé de fabrication d'un pansement selon l'une des Revendications 1 à 6, ***caractérisé en ce qu***'une solution, contenant de la nitroglycérine dans la quantité nécessaire pour l'utilisation envisagée pour le pansement et un agent de réticulation, d'un copolymère acrylateacétate de vinyle fabriqué par polymérisation radicalaire d'un mélange de monomères constitué de 21 à 40 % en poids d'acétate de vinyle, de 55 à 70 % en poids d'un alkylester en C₂₋₈ de l'acide acrylique et de 3 à 10 % en poids d'un hydroxyalkylester en C₂₋₄ de l'acide acrylique, rapportés à 100 % en poids de monomères dans le mélange, est appliquée sur la feuille protectrice du pansement selon l'épaisseur de couche désirée, le solvant ou le mélange de solvants est éliminé en chauffant, et ainsi la réticulation du copolymère particulier acrylate-acétate de vinyle est effectuée, puis la feuille support est appliquée et le pansement est découpé et/ou estampé à la taille souhaitée.

8. Procédé selon la Revendication 7, ***caractérisé en ce que*** le copolymère particulier acrylate-acétate de vinyle, la nitroglycérine et l'agent de réticulation sont dissous ensemble dans mélange de solvants qui contient 20 à 40 % en poids d'éthanol ou d'un mélange d'éthanol et de méthanol, et sa fraction de matières solides, constituée du copolymère particulier acrylate-acétate de vinyle, de l'agent de réticulation et de la nitroglycérine, vaut 40 à 60 % en poids.
